# EUROPEAN PATENT APPLICATION

(11) **EP 1 980 840 A1**
(43) Date of publication of application: **15.10.2008**
(21) Application number: 07706266.9
(22) Date of filing: 16.01.2007
(51) Int. Cl.: G01N 21/59, F16C 19/52

(54) **LUBRICANT DETERIORATION DETECTOR AND BEARING WITH DETECTOR**

(30) Priority: 23.01.2006 JP 2006013468; 23.01.2006 JP 2006013469; 23.01.2006 JP 2006013470; 24.01.2006 JP 2006014597; 30.01.2006 JP 2006021108
(71) Applicant: NTN Corporation, Osaka-shi, Osaka 550-0003 (JP)
(72) Inventor: TAKAHASHI, Toru, Iwata-shi, Shizuoka 4380037 (JP); NAKAJIMA, Akio, Iwata-shi, Shizuoka 4380037 (JP); MAEDA, Kazunari, Iwata-shi, Shizuoka 4380037 (JP); SUZUKI, Kouyou, Iwata-shi, Shizuoka 4380037 (JP)
(74) Representative: Hirsch & Associés
(86) International application number: PCT/JP2007/000014
(87) International publication number: WO 2007/083520

(57) **Abstract**

To provide a lubricant deterioration detecting device capable of detecting the status of deterioration of the lubricant with high accuracy and also to provide a detector equipped bearing assembly capable of detecting the status of deterioration of the lubricant accurately and in real time, the lubricant deterioration detecting device includes a light emitting unit 2 and a light receiving unit 3 arranged on respective sides of a lubricant 5 forming an object to be detected, and a determining circuit 4 for determining the light transmittance of the lubricant 5 in reference to an output from the light receiving unit 3 to thereby detect the amount of foreign matter admixed in the lubricant 5. This lubricant deterioration detecting device is incorporated in a bearing assembly.

## Description

### BACKGROUND OF THE INVENTION

### (Field of the Invention)

The present invention relates to a lubricant deterioration detecting device for detecting the status of deterioration a lubricant resulting from admixture of an alien substance and also to a detecting device incorporated bearing assembly equipped with such lubricant detection detecting device such as, for example, a lubricant deterioration detecting device incorporated bearing assembly for use in railway vehicles, automotive vehicles and industrial machines or the like.

### (Description of the Prior Art)

In the bearing assembly filled with a lubricant, an insufficient lubrication occurs once the lubricant (such as, for example, a grease or oil) within the bearing assembly is deteriorated, resulting in a reduction in lifetime of the bearing assembly. Determination of the occurrence of the insufficient lubrication in reference to the status of vibration occurring in the bearing assembly is carried out when and after an operating abnormality has occurred as a result of expiration of the lifetime of the bearing assembly and is, accordingly, incapable of detecting the presence or absence of an abnormality in lubrication at an early stage. In view of this, it has been desired to monitor the status of the lubricant within the bearing assembly regularly or in real time so that the occurrence of the abnormality and/or the time of maintenance can be predicated.

A major cause of deterioration of the lubricant may be enumerated an admixture of a powdery wear debris, which is produced as the bearing assembly is used, with the lubricant.

For detecting the status of wear of the bearing assembly, a sensor incorporated bearing assembly has been suggested, in which an electrode is arranged inside a sealing member of the bearing assembly so that the electrical characteristic of the lubricant resulting from the admixture of the wear debris can be detected in terms of a change in resistance, electrostatic capacitance, magnetic resistance or impedance. (See, for example, the Japanese Laid-open Patent Publication No. 2004-293776, published October 21, 2004.)

However, since the sensor incorporated bearing assembly disclosed in the above mentioned patent document is so designed that the electrical characteristic of the lubricant can be detected, no change in characteristic can be detected unless a condition is established in which conduction occurs as a result of inclusion of a substantial amount of wear debris and, thus, difficulty often occurs in detecting the inclusion of the alien substance.

### SUMMARY OF THE INVENTION

An object of the present invention is to provide a lubricant deterioration detecting device capable of detecting the status of deterioration of the lubricant with high accuracy and also to provide a detecting device incorporated bearing assembly capable of detecting the status of deterioration of the lubricant accurately and in real time.

A lubricant deterioration detecting device according to the present invention includes a light emitting unit and a light receiving unit so arranged as to define a gap therebetween for accommodating a lubricant forming an object to be detected; and a determining circuit for determining a light transmittance of the lubricant in reference to an output from the light receiving unit to thereby detect a status of deterioration of the lubricant.

According to this construction, rays of light emitted from the light emitting unit transmit through the lubricant and the light receiving unit receives the transmitted light. The intensity of the transmitted light in this case varies depending on the amount of foreign matter admixed in the lubricant, and an output from the light receiving unit varies depending on the intensity of the transmitted light. The determining circuit determines the light transmittance of the lubricant to thereby detect the amount of the foreign matter admixed in the lubricant. By way of example, in the case of the lubricant employed in the bearing assembly, the mixing ratio of the foreign matter such as, for example, powdery wear debris increases as the deterioration proceeds, and the status of deterioration of the lubricant can be detected by detecting the amount of the wear debris admixed. In such case, the lubricant deterioration detecting device detects deterioration of the lubricant in reference to the light transmittance and, therefore, the status of deterioration of the lubricant can be detected accurately.

The lubricant deterioration detecting device according to the present invention may additionally include a set of a light emitting unit and a light receiving unit having a gap therebetween for accommodating a lubricant forming a reference so that the determining circuit can compare respective outputs of two sets of the light emitting unit and the light receiving unit to thereby detect the status of deterioration of the lubricant.

According to this construction, since the light transmittances of the reference lubricant and the lubricant forming the object to be detected are compared with each other, it is possible to further accurately detect the amount of the foreign matter admixed and the status of deterioration of the lubricant. By way of example, even when the characteristic of the light emitting element forming the light emitting unit changed in dependence on temperature, the amount of such change can be counterbalanced by a differential construction of the determining circuit and, therefore, not only can the status of deterioration of the lubricant be detected accurately, but the stabilized detection can also be accomplished.

In the lubricant deterioration detecting device according to the present invention, the light emitting unit may include at least one light emitting element and the light receiving unit may include a plurality of light receiving elements, and one or some of the plural light receiving elements are arranged in a direction, in which rays of light emitted from the light emitting element and subsequently transmitted through the lubricant forming the object to be detected are detected, and the remaining light receiving elements are arranged in a direction, in which the rays of light emitted from the light emitting element and subsequently scattered by the lubricant are captured, and wherein the determining circuit is operable to compare an output or outputs from the light receiving elements, arranged in the direction for detecting the transmitted rays of light, and the remaining light receiving elements to thereby detect the status of deterioration of the lubricant.

According to this construction, the only light receiving element can detect the light transmitted through the lubricant and the other light receiving elements can detect the light scattered from the lubricant, and the detected intensity of the transmitted light and the detected intensity of the scattered light are compared with each other by the determining circuit to thereby detect the proportion of the alien substance in the lubricant and the status of deterioration of the lubricant. In such case, since the intensity of the transmitted light and the intensity of the scattered light are compared with each other, the difference in optical characteristic can be detected and, therefore, detailed information on deterioration such as, for example, coloring can be obtained, allowing the kind of the foreign matter admixed in the lubricant to be estimated.

In the lubricant deterioration detecting device according to the present invention, the light emitting unit may include a plurality of light emitting elements operable to emit respective rays of light having different wavelengths and the light receiving unit may include a light receiving elements for receiving rays of light from the plural light emitting elements and wherein the determining circuit is operable to detect the status of deterioration of the lubricant on the basis of a difference in absorptivity for each of the wavelengths detected from an output of the light receiving element.

With the lubricant deterioration detecting device so constructed as hereinabove described, the rays of light emitted from the plural (for example, two) light emitting elements and subsequently transmitted through the lubricant can be detected by the light receiving element. In such case, when the light emitting elements are alternately lit, the light receiving element measures, on a time sharing basis, the light transmittance (or light absorptivity) for each of wavelengths at the lubricant and, therefore, the intensity of the transmitted light for each wavelength can be detected. Since the intensity of the transmitted light decreases as the amount of the foreign matter such as, for example, iron powder contained in the lubricant forming the object to be detected increases, the determining circuit can estimate the amount of the foreign matter in the lubricant from the detected intensity of the transmitted light.

Where the lubricant is used in, for example, the bearing assembly, a major cause of deterioration of the lubricant may be an admixture of a powdery wear debris, which is produced as the bearing assembly is used, into the lubricant and, accordingly, by estimating the amount of the wear debris, which is foreign matter admixed into the lubricant, by the determining circuit, the status of deterioration of the lubricant can be estimated. In particular, since in this lubricant deterioration detecting device, the respective intensities of the transmitted light of the different wavelength are detected by the optical system, it is possible to acquire detailed information on the deterioration such as the degree of contamination or coloring of the lubricant and identification of the kind of the foreign matter admixed, from the difference in intensity of the transmitted light according to the wavelength.

In the lubricant deterioration detecting device according to the present invention, the light emitting unit may include at least one light emitting element and the light receiving unit may include a plurality of light receiving elements having different wavelength sensitivities and capable of receiving rays of light from the light emitting element. In this case, the determining circuit is operable to detect the status of deterioration of the lubricant on the basis of the difference in absorptivity of the wavelengths detected by comparing respective outputs of the light receiving elements.

In the lubricant deterioration detecting device so constructed as described hereinabove, the rays of light emitted from the light emitting element and subsequently transmitted through the lubricant can be detected by the plural light receiving elements having different wavelength sensitivities. The comparing circuit can estimate the amount of the foreign matter contained in the lubricant form the detected intensities of the transmitted light and the status of deterioration of the lubricant can be estimated from the estimated amount of the foreign matter. Also, since arrangement is so made that the intensities or light absorptivity of the transmitted light of different wavelengths can be detected by the optical system, detailed information on the deterioration such as the degree of contamination or coloring of the lubricant and identification of the kind of the foreign matter admixed can be acquired from the difference in intensity of the transmitted light according to the wavelength.

In the lubricant deterioration detecting device according to the present invention, a light emitting optical fiber having one end confronting the lubricant and a light receiving optical fiber having one end confronting the lubricant may be employed, in which case the light emitting unit and the light receiving unit are connected respectively with other ends of the light emitting optical fiber and receiving optical fiber.

In the lubricant deterioration detecting device so constructed as described hereinabove, the rays of light emitted from the light emitting unit are, after having travelled through the light emitting optical fiber, transmitted through the lubricant and are subsequently detected by the light receiving units after having travelled through the light receiving optical fiber. The intensity of the transmitted light transmitted through the lubricant in this way decreases as the amount of the foreign matter contained in the lubricant increases, and, accordingly, the determining circuit can estimate the amount of the foreign matter in the lubricant from the intensity of the transmitted light detected by the light receiving units. For example, where in the case of the lubricant used in the bearing assembly, since the mixing proportion of the foreign matter such as, for example, the powdery wear debris increases as the deterioration proceeds, the status of deterioration of the lubricant can be accurately detected if the amount of the foreign matter admixed in the lubricant within the bearing assembly is detected with the lubricant deterioration detecting device.

In particular, since in this lubricant deterioration detecting device, respective ends of the light emitting and receiving optical fibers are held in face-to-face relation with the lubricant forming the object to be detected and the other ends of those optical fibers are connected respectively with the light emitting unit and the light receiving unit, the light emitting unit, the light receiving unit and the determining circuit can be arranged separated from the detecting section and, therefore, an electronic circuit or the like including the light receiving unit and the determining circuit can stably detect the status of deterioration of the lubricant without being adversely affected by electrical noises and/or temperature dependent change.

Also, since a detecting section can be constructed compactly, where the use is made to detect the deterioration of, for example, the lubricant sealed within the bearing assembly, the detecting section can be simply and compactly installed inside the bearing assembly. For this reason, the detecting section does not constitute a cause of interference with the flow of the lubricant and the lubricant can be stably supplied to the detecting section, thus increasing the flexibility of arrangement of the detecting section inside the bearing assembly.

Yet, since the respective ends of the light emitting and receiving optical fibers form parts of the detecting section of the lubricant, the sectional surface area of a measuring site of the lubricant can be minimized and even the lubricant having a high viscosity can readily enter the detecting section, with the detection consequently stabilized.

In the lubricant deterioration detecting device according to the present invention, one or both of the optical fibers may be provided with a lens at one end thereof. Where the lens is provided at one end of the optical fiber in this way, it is possible to avoid scattering of light and, therefore, not only can the site of measurement on the lubricant be miniaturized, but also the detecting sensitivity can be increased.

In the lubricant deterioration detecting device according to the present invention, the end of one or both of the optical fibers, which is on the side confronting the lubricant, may confront the lubricant via a mirror. In the case of this construction, the end of the optical fiber can assuredly confront the lubricant without being limited by the direction of arrangement of the optical fiber and, therefore, the freedom of arrangement in, for example, the bearing assembly can be increased.

A lubricant deterioration detecting device incorporated bearing assembly in accordance with the first aspect of the present invention is a bearing assembly having the lubricant deterioration detecting device, mounted thereon, of the invention described above.

According to this construction, with the lubricant deterioration detecting device of the present invention, the proportion of the alien substance within the lubricant can be detected from the light transmittance and the status of the lubricant can be monitored in real time. For this reason, the necessity of replacement of the lubricant can be determined prior to an actual occurrence of an operating abnormality and any damage to the bearing assembly resulting from the failure of the lubricant can be avoided. Also, since the necessity of replacement of the lubricant can be determined in reference to the sensor output, the amount of the lubricant to be discarded earlier than the lifetime of use can be reduced.

A lubricant deterioration detecting device incorporated bearing assembly in accordance with the second aspect of the present invention is the one having the lubricant deterioration detecting device, mounted thereon, of the above described invention including the optical fibers.

According to this construction, it is possible to accurately detect the deterioration of the lubricant, which is sealed within the bearing assembly, regularly and in real time. Accordingly, the necessity of replacement of the lubricant can be determined prior to an actual occurrence of an operating abnormality and any damage to the bearing assembly resulting from the failure of the lubricant can be avoided. Also, since the necessity of replacement of the lubricant can be determined in reference to the output of the lubricant deterioration detecting device, the amount of the lubricant to be discarded earlier than the lifetime of use can be reduced.

In the second lubricant deterioration detecting device incorporated bearing assembly of the present invention, the light emitting unit and the light receiving unit may be arranged inside or outside the bearing assembly. Where it is arranged outside the bearing assembly, only the detecting section is arranged inside the bearing assembly and, therefore, the construction within the bearing assembly can be downsized.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a diagram showing a principle construction of a lubricant deterioration detecting device according to a first preferred embodiment of the present invention;
Fig. 2 is a detailed structural diagram showing the lubricant deterioration detecting device according to the first embodiment;
Fig. 3 is an explanatory diagram showing an example of arrangement of a light emitting element and a light receiving element both employed in the lubricant deterioration detecting device according to the first embodiment;
Fig. 4 is a chart showing the relation between the proportion of an alien substance contained in the lubricant and the ratio of the intensity of light transmitted at that time;
Fig. 5 is a diagram showing a basic structure of the lubricant deterioration detecting device according to a second preferred embodiment of the present invention;
Fig. 6 is a schematic structural diagram showing the lubricant deterioration detecting device according to a third preferred embodiment of the present invention;
Fig. 7 is a circuit diagram showing a light receiving unit and a determining circuit both employed in the lubricant deterioration detecting device according to the third embodiment;
Fig. 8 is a schematic structural diagram showing the lubricant deterioration detecting device according to a fourth preferred embodiment of the present invention;
Fig. 9 is a schematic structural diagram showing the lubricant deterioration detecting device according to a fifth preferred embodiment of the present invention;
Fig. 10 is a schematic structural diagram showing the lubricant deterioration detecting device according to a sixth preferred embodiment of the present invention;
Fig. 11 is a schematic structural diagram showing the lubricant deterioration detecting device according to a seventh preferred embodiment of the present invention;
Fig. 12 is a schematic structural diagram showing the lubricant deterioration detecting device according to an eighth preferred embodiment of the present invention;
Fig. 13 is a schematic structural diagram showing the lubricant deterioration detecting device according to the eighth embodiment;
Fig. 14 is a schematic structural diagram showing the lubricant deterioration detecting device according to a ninth preferred embodiment of the present invention;
Fig. 15 is a schematic structural diagram showing the lubricant deterioration detecting device according to a tenth preferred embodiment of the present invention;
Fig. 16 is a schematic structural diagram showing the lubricant deterioration detecting device according to an eleventh preferred embodiment of the present invention;
Fig. 17 is a schematic structural diagram showing the lubricant deterioration detecting device according to a twelfth preferred embodiment of the present invention;
Fig. 18 is a schematic structural diagram showing the lubricant deterioration detecting device according to a thirteenth preferred embodiment of the present invention;
Fig. 19 is a sectional view showing an example of a detecting device incorporated bearing assembly having the lubricant deterioration detecting device mounted thereon;
Fig. 20 is a sectional view showing another example of the detecting device incorporated bearing assembly having the lubricant deterioration detecting device mounted thereon;
Fig. 21 is a sectional view showing an example of the lubricant deterioration detecting device incorporated bearing assembly having the lubricant deterioration detecting device according to the eighth embodiment;
Fig. 22 is an enlarged diagram showing a portion indicated by A in Fig. 21;
Fig. 23 is a fragmentary enlarged sectional view showing an example of the lubricant deterioration detecting device incorporated bearing assembly having the lubricant deterioration detecting device according to the embodiment shown in Fig. 18;
Fig. 24 is a block diagram showing a principle construction of the lubricant deterioration detecting device according to a first specific example of an applied technology, which has a fundamental construction different from that of the present invention;
Fig. 25 is a sectional view showing the lubricant deterioration detecting device according to the first specific example of the applied technology;
Fig. 26A is a fragmentary plan view showing an example of a second detecting section used in the lubricant deterioration detecting device according to the first specific example of the applied technology;
Fig. 26B is a cross-sectional view taken along the line III-III in Fig. 26A;
Fig. 27 is a plan view showing another example of the second detecting section employed in the lubricant deterioration detecting device according to the first specific example of the applied technology;
Fig. 28A is a plan view showing a schematic construction of the lubricant deterioration detecting device according to a second specific example of the applied technology;
Fig. 28B is a front elevational view of the lubricant deterioration detecting device according to the second specific example of applied technology;
Fig. 29 is a sectional view showing an example of the detector equipped bearing assembly having mounted thereon the lubricant deterioration detecting device according to the second specific example of the applied technology; and
Fig. 30 a sectional view showing another example of the detector equipped bearing assembly having mounted thereon the lubricant deterioration detecting device according to the second specific example of the applied technology.

### DETAILED DESCRIPTION OF THE EMBODIMENTS

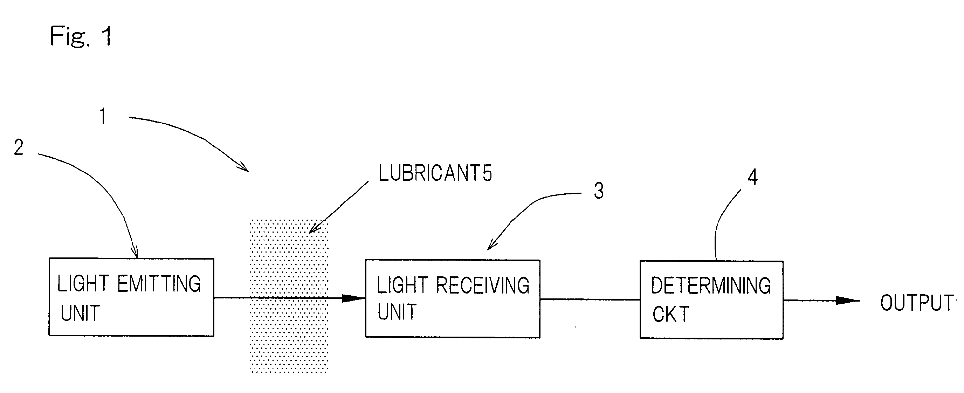

A first preferred embodiment of the present invention will be described with particular reference to Figs. 1 to 4. Fig. 1 illustrates a diagram showing the principle construction of the lubricant deterioration detecting device according to this first embodiment. This lubricant deterioration detecting device 1 includes a light emitting unit 2 and a light receiving unit 3 defining a gap therebetween for accommodating a lubricant 5, which forms an object to be detected, intervening therebetween, and a circuit in the form of a determining circuit 4 for determining the light transmittance of the lubricant 5 in reference to an output of the light receiving unit 3 to thereby detect the amount of alien substances admixed in the lubricant 5. The light emitting unit 2 includes a light emitting element 6 such as, for example, a light emitting diode and the light receiving unit 3 includes a light receiving element 8 such as, for example, a phototransistor.

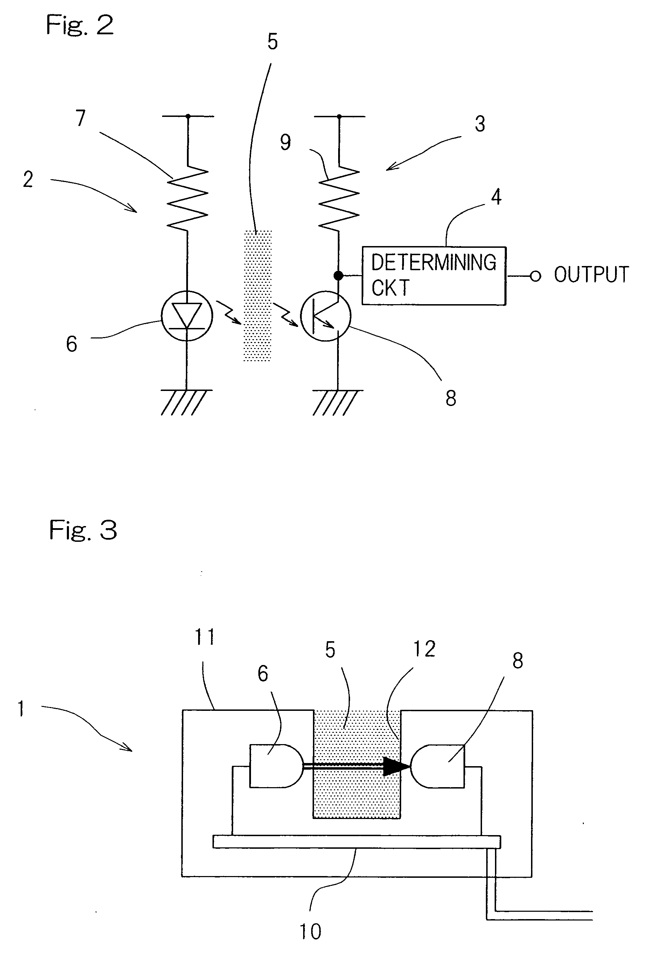

Fig. 2 illustrates an example of a specific construction of the lubricant deterioration detecting device 1. In this figure, the light emitting unit 2 includes a light emitting element 6 (a light emitting diode in this embodiment) and a resistor 7 connected in series between an electric power source and the ground. The light receiving unit 3 includes a light receiving element 8 (a phototransistor in this embodiment) and a resistor 9 connected in series between an electric power source and the ground. Accordingly, rays of light emitted from the light emitting element pass through the lubricant 5 and are then detected by the light receiving element 8. A circuit in the form of the determining circuit 4, which is connected between the light receiving element 8 and the resistor 9, is operable to detect a photoelectric current flowing through the light receiving element 8. In this construction, the light emitting element 6 and the light receiving element 8 may be employed in the form of a photo-interrupter employing a set of light emitting and receiving elements.

Fig. 3 illustrates an example of arrangement of the light emitting element 6 and the light receiving element 8 both employed in the lubricant deterioration detecting device 1 shown in and described with reference to Fig. 2. In this figure, the light emitting unit 2, the light receiving unit 3 and the circuit forming the determining circuit 4 are mounted on a circuit substrate 10, which is in turn arranged within a housing 11. The housing 11 has a gap 12 capable of accommodating a lubricant oil 5, which forms an object to be detected, and the light emitting element 6 and the light receiving element 8 are arranged so as to confront each other on the same axis with the gap 12 intervening therebetween.

Although only the contour of the housing 11 is shown in Fig. 3, the housing 11 may be in the form of a hollow casing or in the form of a resin molding in which the circuit substrate 10, the light emitting element 6 and the light receiving element 8 are integrated together.

According to the lubricant deterioration detecting device 1 of the construction described hereinabove, light from the light emitting element 6 of the light emitting unit 2 is transmitted through the lubricant 5 and the light receiving element 8 of the light receiving unit 3 receives the light which has so transmitted. The intensity of the transmitted light in this case varies depending on the amount of an alien substance contained in the lubricant 5 and the photocurrent flowing across the light receiving element 8 as a result of receipt of the transmitted light varies correspondingly depending on the variation of the intensity of the transmitted light. The determining circuit 4 determines the light transmittance of the lubricant 5 in reference to an output from the light receiving element 8, thereby detecting the amount of the alien substance mixed in the lubricant 5.

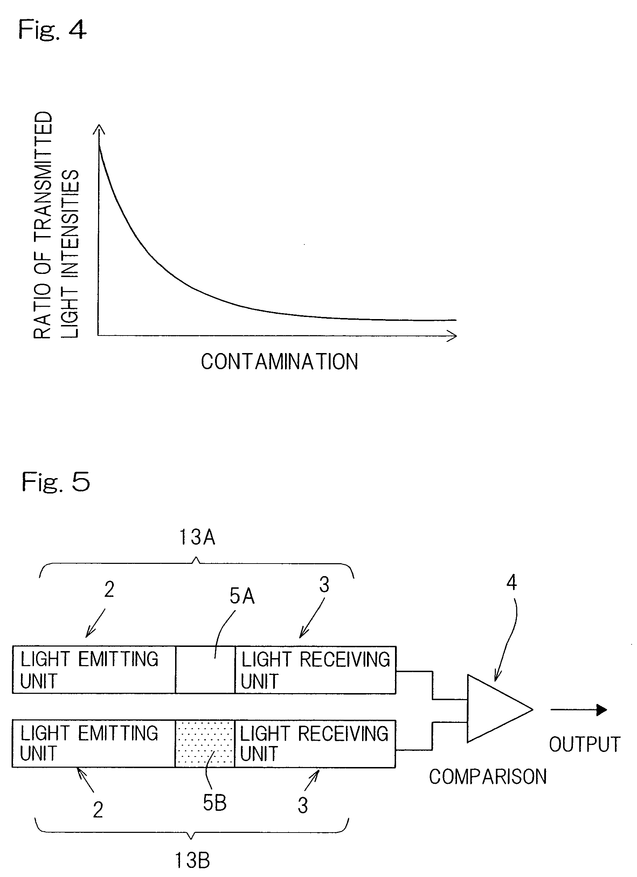

Fig. 4 illustrates a chart descriptive of the proportion of the relation between the alien substance contained in the lubricant 5 and the ratio of the intensity of the transmitted light. According to this chart, it can readily be seen that there is such a relationship that the ratio of the intensity of the transmitted light decreases with increase of the alien substance. In view of this, the amount of the foreign matter mixed in the lubricant 5 can be detected with the lubricant deterioration detecting device 1 referred to above. In the case of the lubricant of a kind used in a bearing assembly, the proportion of the foreign matter such as, for example, a powdery wear debris, increases as deterioration proceeds and, accordingly, by detecting the amount of the foreign matter mixed in the lubricant within the bearing assembly with the lubricant deterioration detecting device 1, the status of deterioration of the lubricant can be detected accurately.

Fig. 5 illustrates a diagram showing the principle construction according to a second preferred embodiment of the present invention. In the lubricant deterioration detecting device 1 according to this embodiment, two light emitting and receiving sets 13A and 13B, each set consisting of a light emitting unit 2 and a light receiving unit 3, are employed. In this embodiment, a lubricant 5A forming a reference is intervened between the light emitting unit 2 and the light receiving unit 3 of one set 13A of the light emitting and receiving sets 13A and 13B and a lubricant 5B forming the object to be detected is intervened between the light emitting unit 2 and the light receiving unit 3 of the other set 13B of the light emitting and receiving sets 13A and 13B. The determining circuit 4 is made up of a differential circuit for comparing respective outputs from the light receiving units 3 of the two light emitting and receiving sets 13A and 13B and the amount of the foreign matter mixed in the lubricant 5B forming the object to be detected is detected through the comparison performed thereby. The light emitting unit 2 and the light receiving unit 3 of each of those sets are similar in structure to those shown and described in connection with the first embodiment.

In the case of the lubricant deterioration detecting device of the construction described above, since the light transmittance of the reference lubricant and that of the lubricant forming the object to be detected are compared with each other, the amount of the foreign matter mixed and the status of deterioration of the lubricant can be detected further accurately and stably. By way of example, even though the characteristic of the light receiving element forming a part of the light receiving unit 2 changes with change of temperature, the amount of change can be counterbalanced by the differential construction and, therefore, not only can the status of deterioration of the lubricant be detected accurately, but a stable detection can also be achieved.

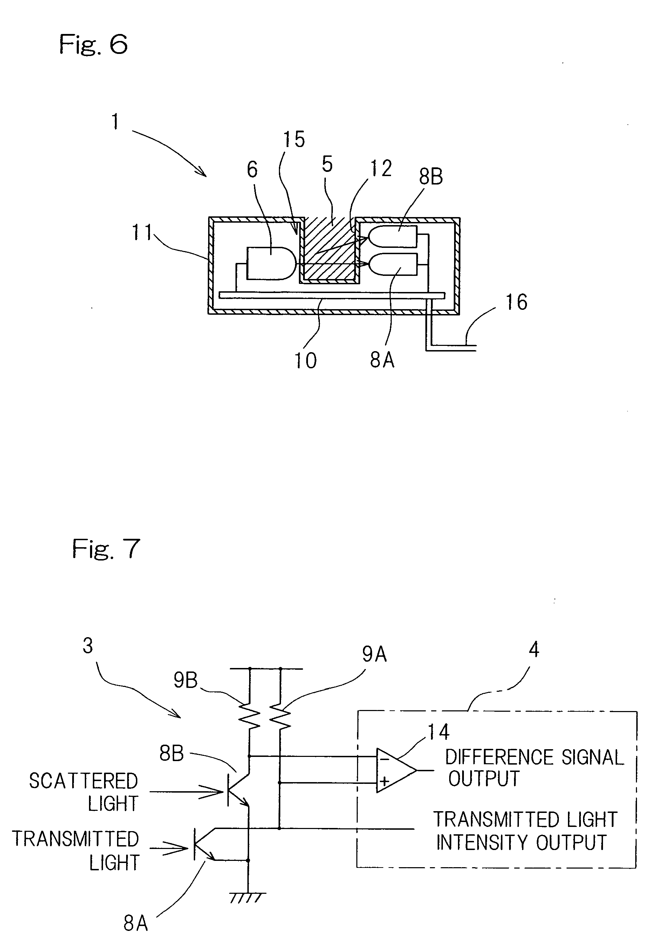

A third preferred embodiment of the present invention will now be described with particular reference to Figs. 6 and 7. Fig. 6 illustrates a schematic structural diagram showing the lubricant deterioration detecting device 1 according to this embodiment. This lubricant deterioration detecting device 1 includes an optical system 15, made up of a light emitting element 6 and a plurality of (for example, two, in this instance) light receiving elements 8A and 8B, and a determining circuit 4 (Fig. 7). One of the plural light receiving elements 8A and 8B (for example, the light receiving element 8A) is arranged in such a direction as to allow it to detect a transmitted light, which is emitted from the light receiving element 6 and then transmitted through the lubricant 5 forming the object to be detected. The other light receiving element 8B is arranged in such a direction as to allow it to pick up a scattered light which is emitted from the light emitting element 6 and then scattered by the lubricant 5. The other light receiving element 8B is arranged, for example, in parallel with the light receiving element 8A that detects the transmitted light. The light emitting element 6, the light receiving elements 8A and 8B and the determining circuit 4 are mounted on a common circuit substrate 10, which is in turn installed within a housing 11. The housing 11 has a gap 12 defined by, for example, a recess capable of accommodating therein the lubricant 5 forming the object to be detected and the light emitting element 6 and the light receiving elements 8A and 8B are positioned on respective sides of the gap 12 so as to confront with each other. The determining circuit 4 is of a type capable of comparing an output from the light receiving element 8A, arranged in such a direction as to detect the transmitted light, and an output from the light receiving element 8B, arranged in such a direction as to pick up the scattered light, with each other so that in accordance with a preset reference, the status of deterioration of the lubricant 5 can be detected. This determining circuit 4 output a detection signal to the outside through a wiring cable 16. Also, through this wiring cable 16, an electric power is supplied from the outside to the lubricant deterioration detecting device 1.

Fig. 7 illustrates an example of a circuit construction including the light receiving unit 3, made up of the light receiving elements 8A and 8B, and the determining circuit 4 provided in the subsequent stage thereof. In this figure, the light receiving unit 3 includes the light receiving element 8A such as, for example, a phototransistor and a light receiving side resistor 9A, both connected in series between an electric power source and the ground, and the light receiving element 8B such as, for example, a phototransistor and a light receiving side resistor 9B both connected in series between the electric power source and the ground. The determining circuit 4 includes a differential amplifier circuit 14 which is supplied with a photoelectric current signal corresponding to the intensity of the transmitted light, which is outputted from a junction between the light receiving element 9A and the light receiving side resistor 9A, and a photoelectric current signal corresponding to the intensity of the scattered light, which is outputted from a junction between the light receiving element 8B and the light receiving side resistor 9B, and capable of outputting a difference signal of those photoelectric current signals, that is, a signal indicative of the difference between the intensity of the transmitted light and the intensity of the scattered light. It is to be noted that in such case, the determining circuit 4 is also supplied with a single photoelectric current signal from the light receiving element 8A, which corresponds to the intensity of the transmitted light.

With the lubricant deterioration detecting device 1 of the construction described above, the transmitted light across the lubricant 5 and the scattered light from the lubricant 5 can be detected by the light receiving element 8A and the light receiving element 8B, respectively, and the intensity of the transmitted light so detected and the intensity of the scattered light so detected are compared by the determining circuit to detect the status of deterioration of the lubricant 5. Considering that where the lubricant 5 forming the object to be detected is nearly transparent, the intensity of the transmitted light becomes higher, but where the lubricant 5 is thick with containant, the intensity of the scattered light increases, comparison of the intensity of the detected transmitted light and the intensity of the detected scattered light with each other by means of the determining circuit 4 is effective to provide detailed information on the deterioration such as the degree of contamination or coloring of the lubricant 5. By way of example, it is also possible to estimate the kind of the foreign matter admixed in the lubricant 5. It is to be noted that in this embodiment, it is possible to estimate the status of deterioration of the lubricant from a single signal inputted to the determining circuit 4, which is indicative of the intensity of the transmitted light.

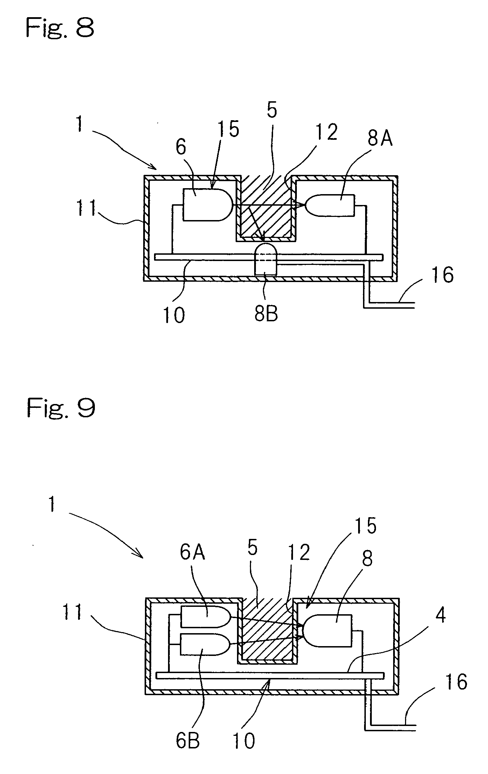

Fig. 8 illustrates a schematic structural diagram showing a fourth preferred embodiment of the present invention. The lubricant deterioration detecting device 1 according to this embodiment is similar to that according to the third embodiment shown in and described with reference to Fig. 6, but differs therefrom in that the light receiving element 8B for detecting the scattered light is so arranged as to be oriented in a direction generally perpendicular to the optical axis of the light receiving element 8A for detecting the transmitted light. Other structural features thereof than those described above are similar to those shown and described in connection with the third embodiment.

Other than that described above, the light receiving element 8B for detecting the scattered light may be arranged in any pattern, for example, may be oriented in a direction at an angle of 45° relative to the optical axis of the light receiving element 8A for detecting the transmitted light and may accordingly be arranged at a suitable position in consideration with the characteristic of the lubricant 5 forming the object to be detected.

Although in any one of the foregoing embodiments, reference has been made to the only light receiving unit 3 including a plurality of light receiving elements 8A and 8B, a plurality of light receiving units 3 may be arranged being distributed to a corresponding number of positions. Also, a plurality of light receiving units 3 having respective different sensitivities to wavelength may be arranged.

A fifth preferred embodiment of the present invention will be described with particular reference to Fig. 9. Fig. 9 illustrates a schematic structural diagram of the lubricant deterioration detecting device 1 according to this embodiment. This lubricant deterioration detecting device 1 includes an optical system 15, made up of a plurality of (for example, two, in this embodiment) light emitting elements 6A and 6B operable to emit respective lights of different wavelengths, a single light receiving element 8 for receiving rays of light emitted from those plural light emitting elements 6A and 6B, and a determining circuit 4. For the light emitting elements 6A and 6B, light emitting diodes such as, for example, a red LED and a blue LED are employed, respectively. For the light receiving element 8, a light receiving element sensitive to the wavelengths of light from both of the light emitting elements 6A and 6B may be selected. The optical system 15 referred to above has the lubricant 5, forming an object to be detected, disposed on the optical path between the plural light emitting elements 6A and 6B and the light receiving element 8. The light emitting elements 6A and 6B, the light receiving element 8 and the determining circuit 4 are mounted on a circuit substrate 10, which is in turn installed within a housing 11. The housing 11 has a gap 12 such as, for example, a recess capable of accommodating therein the lubricant 5 forming the object to be detected and the light emitting elements 6A and 6B and the light receiving element 8 are positioned on respective sides of the gap 12 so as to confront with each other. The determining circuit 4 is of a type capable of utilizing a difference in wavelength absorptivity, outputted from the light receiving element 8, to thereby estimate the status of deterioration of the lubricant 5 according to a preset reference. A detection signal emerging from the determining circuit 4 is supplied to the outside through a wiring cable 16. Also, through this wiring cable 16, an electric power is supplied from the outside to the lubricant deterioration detecting device 1.

For the light emitting elements 6A and 6B, other than an LED, an incandescent bulb, a semiconductor laser diode, an EL, an organic EL or a fluorescent tube may be employed. Also, for the light receiving element 8, a photodiode, a phototransistor, a CDS, a solar cell or a photomultiplier tube may be employed.

According to the lubricant deterioration detecting device 1 of the construction described above, rays of light emitted from the plural light emitting elements 6A and 6B and subsequently transmitted through the lubricant 5 are detected by the single light receiving element 8. In this case, the light emitting elements 6A and 6B are to be alternately lit. In this way, the light receiving element 8 alternately detects light of a predetermined wavelength, which has been emitted from one 6A of the light emitting elements and subsequently transmitted through the lubricant 5, and light of a predetermined wavelength different from the above described wavelength, which has been emitted from the other 6B of the light emitting elements and subsequently transmitted through the lubricant 5. In other words, the light receiving element 8 measures on a time sharing basis, the light transmittance (or light absorptivity) of each wavelength across the lubricant 5 and is, hence, capable of detecting the amount of light of each wavelength which has been transmitted through the lubricant 5. The larger the amount of the foreign matter such as, for example, iron powder contained in the lubricant 5 forming the object to be detected, the lower the intensity of the transmitted light and, accordingly, the determining circuit 4 can estimate the amount of the foreign matter in the lubricant 5 in reference to the detected intensity of the transmitted light.

Where the lubricant 5 is used in, for example, a bearing assembly, a major cause of deterioration of the lubricant 5 may be an admixture of powdery wear debris, which is produced as the bearing assembly is used, into the lubricant 5. Accordingly, by estimating the amount of the wear debris, which is foreign matter admixed into the lubricant 5, by the determining circuit 4, the status of deterioration of the lubricant 5 can be estimated. In particular, since in the lubricant deterioration detecting device 1, the respective intensities of the transmitted light of the different wavelength are detected by the optical system 15, it is possible to acquire detailed information on the deterioration such as the degree of contamination or coloring of the lubricant 5 and identification of the kind of the foreign matter admixed, from the difference in intensity of the transmitted light according to the wavelength.

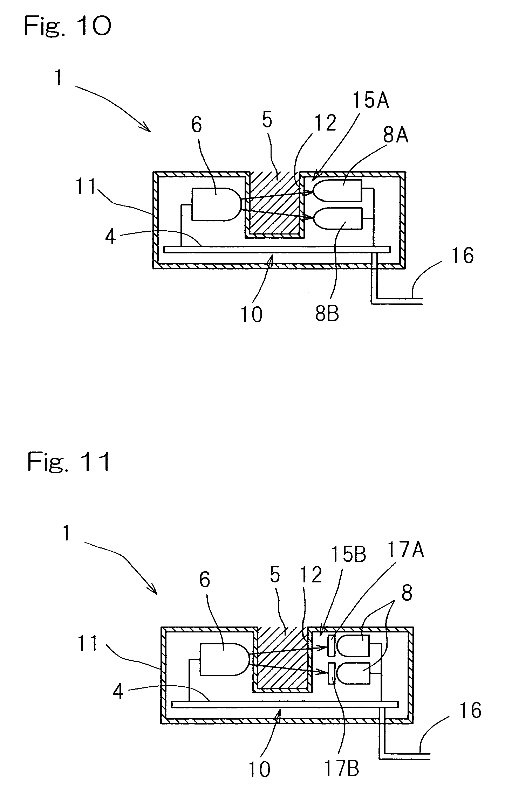

Fig. 10 illustrates a schematic structural diagram showing a sixth preferred embodiment of the present invention. The lubricant deterioration detecting device 1 according to this embodiment includes an optical system 15A, made up of a single light emitting element 6 and a plurality of (for example, two, in this instance) light receiving elements 8A and 8B of respective different wavelength sensitivities for receiving light from the single light emitting element 6, and a determining circuit 4. One light receiving element 8A of the light receiving elements is employed in the form of a light receiving element sensitive to, for example, a red color and the other light receiving element 8B of the light receiving elements is employed in the form of a light receiving element sensitive to, for example, a blue color. For the light emitting element 6, a light emitting element capable of emitting light, for example, a white light having a wavelength range containing respective wavelengths of the red and blue colors is selected. The optical system 15A referred to above has the lubricant 5, forming an object to be detected, disposed on the optical path between the light emitting element 6 and the light receiving elements 8A and 8B. In the sixth embodiment, similarly to the case of the fifth embodiment, the light emitting element 6, the light receiving elements 8A and 8B and a circuit forming the determining circuit 4 are mounted on a circuit substrate 10, which is in turn installed within a housing 11, the housing 11 has a gap 12 capable of accommodating therein the lubricant 5 forming the object to be detected and the light emitting element 6 and the light receiving elements 8A and 8B are positioned on respective sides of the gap 12 so as to confront with each other, are identical with those in the fifth embodiment. The determining circuit 4 is of a type capable of comparing the respective outputs from the light receiving elements 8A and 8B to estimate the status of deterioration of the lubricant 5 in accordance with the preset reference from the difference in transmittance (or light absorptivity) of the light of each wavelength that is detected. In a manner similar to that in the fifth embodiment, a detection signal emerging from the determining circuit 4 is supplied to the outside through a wiring cable 16 and, also, an electric power is supplied from the outside to the lubricant deterioration detecting device 1 through this wiring cable 16.

In the lubricant deterioration detecting device 1 of the construction described above, rays of light emitted from the light emitting element 6 and subsequently transmitted through the lubricant 5 are detected by the light receiving elements 8A and 8B having different sensitivities to wavelength. In other words, the light receiving elements 8A and 8B detect respective intensities of the transmitted light of different wavelengths, which has been emitted from the light emitting element 6 and subsequently transmitted through the lubricant 5. The determining circuit 4 can estimate the amount of the foreign matter contained in the lubricant form the detected intensities of the transmitted light.

Even with this lubricant deterioration detecting device 1, it is possible to estimate the status of deterioration of the lubricant 5 by estimating the amount of the foreign matter admixed in the lubricant 5 with the determining circuit 4. Also, since the intensities (or absorptivities) of the transmitted light of different wavelengths are detected by the optical system 15A, detailed information on the deterioration such as the degree of contamination or coloring of the lubricant 5 and identification of the kind of the foreign matter admixed can be acquired from the difference in intensity of the transmitted light according to the wavelength.

Fig. 11 illustrates a schematic structural diagram showing a seventh preferred embodiment of the present invention. The lubricant deterioration detecting device 1 according to this embodiment is similar to that according to the embodiment shown in and described with reference to Fig. 10, but differs therefrom in that in place of the light receiving elements 8A and 8B sensitive to the different wavelengths, a plurality of common light receiving elements 8 having a broad wavelength sensitivity is employed and, also, filters 17A and 17B having different wavelength characteristics are arranged in front of those light receiving elements 8. One filter 17A of the filters is of a kind capable of transmitting, for example, a red light therethrough while the other filter 17B of the filters is of a kind capable of transmitting, for example, a blue light therethrough. In this way, a combination of the filter 17A and the light receiving element 8 has a function equivalent to that of the light receiving element 8A in Fig. 10 and a combination of the filter 17B and the light receiving element 8 has a function equivalent to that of the light receiving element 8B in Fig. 10. Other structural features than those described above are similar to those in the embodiment shown in and described with reference to Fig. 10.

With the lubricant deterioration detecting device 1 of the construction described above, rays of light emitted from the single light emitting element 6 and subsequently transmitted through the lubricant 5 are detected by the respective light receiving elements 8 through the filters 17A and 17B having the different wavelength characteristics. In this way, the light receiving elements 8 detect respectively the intensities of light, emitted from the light emitting element 6 and subsequently transmitted through the lubricant 5, but having different wavelengths. The determining circuit 4 can estimate the amount of the foreign matter in the lubricant 5 from the detected intensities of the transmitted light. Also, it is possible to estimate the status of deterioration of the lubricant by estimating the amount of the foreign matter admixed in the lubricant 5 with the determining circuit 4. Even in this case, since the intensities (or absorptivities) of the transmitted light of different wavelengths are detected by the optical system 15A, detailed information on the deterioration such as the degree of contamination or coloring of the lubricant 5 and identification of the kind of the foreign matter admixed can be acquired from the difference in intensities of the transmitted light according to the wavelength.

In the case of this embodiment, since the use is made of the filters 17A and 17B having the different wavelength characteristic so that the light receiving elements 8 can detect the respective intensities of the transmitted light of different wavelengths, a combination of the wavelengths to be detected can easily be altered to suit to the characteristic of the lubricant 5 forming the object to be detected and, in particular, it is possible to employ infrared rays of light of a long wavelength excellent in transmittance.

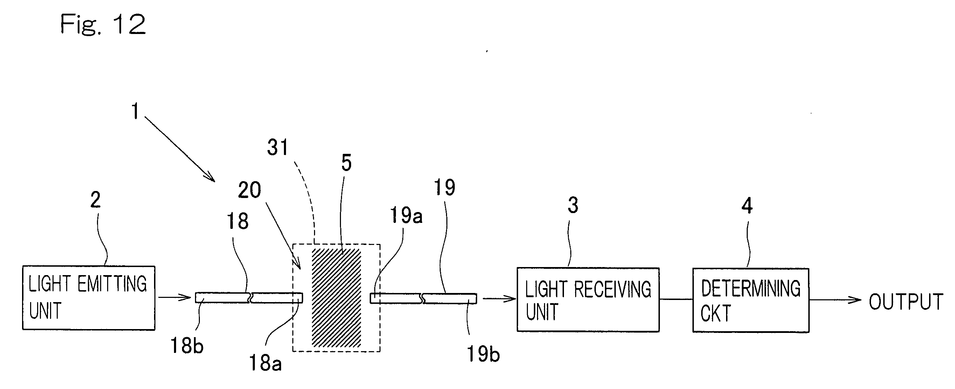

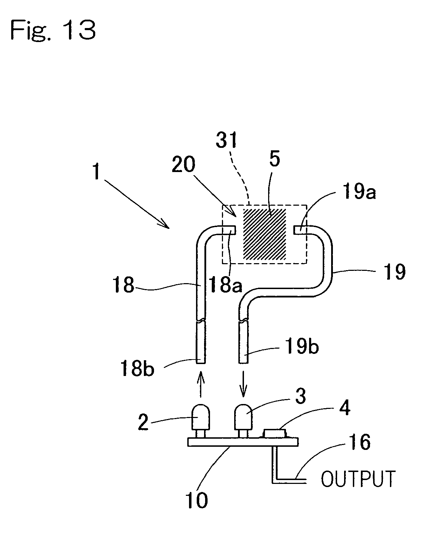

An eighth preferred embodiment of the present invention will now be described with particular reference to Figs. 12 and 13. Fig. 12 illustrates a diagram showing the principle construction according to the eighth preferred embodiment of the present invention. This lubricant deterioration detecting device 1 includes light emitting and receiving optical fibers 18 and 19 having their ends 18a and 19a confronting the lubricant 5 forming an object to be detected, light emitting and receiving units 2 and 3 connected to opposite ends 18b and 19b of those light emitting and receiving optical fibers 18 and 19, and a determining circuit 4 for detecting deterioration of the lubricant 5 from an output of the light receiving unit 3. This determining circuit 4 is in the form of an electronic circuit or the like. The lubricant 5 forming the object to be detected is a lubricant filled within, for example, a bearing assembly 31. The respective ends 18a and 19a of the light emitting and receiving optical fibers 18 and 19, both of which confront the lubricant 5, are so positioned as to assume respective locations on opposite sides of the lubricant 5 so as to confront with each other with the lubricant 5 intervening therebetween.

Fig. 13 illustrates a schematic structural diagram showing the lubricant deterioration detecting device 1 referred to above. The light emitting unit 2 includes a light emitting element such as, for example, an LED having a light emitting surface, in front of which the end 18b of the light emitting optical fiber 18 is so positioned as to confront such light emitting surface. The light receiving unit 3 includes a light receiving element such as, for example, a photodiode having a light receiving surface, in front of which the end 19b of the light receiving optical fiber 19 is so positioned as to confront such light receiving surface. The light emitting unit 2, the light receiving unit 3 and a circuit forming the determining circuit 4 are mounted on the common circuit substrate 10. A detection signal from the determining circuit 4 is outputted to the outside of the device through a wiring cable 16. An electric power is supplied from the outside to the lubricant deterioration detecting device 1 also through the wiring cable 16.

In this lubricant deterioration detecting device 1, the respective ends 18a and 19a of the light emitting and receiving optical fibers 18 and 19, which are so positioned as to confront the lubricant 5, respectively, form a detecting section 20. ' Where this lubricant deterioration detecting device 1 is mounted on a bearing assembly, the detecting section 20 is arranged in the bearing assembly, and portions excluding the detecting section 20, that is, the ends 18b and 19b of the optical fibers 18 and 19, the light emitting unit 2, the light receiving unit 3 and the determining circuit 4 can be positioned outside the bearing assembly.

For the light emitting element forming a part of the light emitting unit 2, other than an LED, an incandescent bulb, a semiconductor laser diode, an EL, an organic EL or a fluorescent tube may be employed. Also, for the light receiving element forming a part of the light receiving unit 3, other than a photodiode, a phototransistor, a CDS, a solar cell or a photomultiplier tube may be employed.

According to the lubricant deterioration detecting device 1 of the construction described above, rays of light emitted from the light emitting unit 2 are transmitted through the lubricant 5 within the bearing assembly 31 by way of the light emitting optical fiber 18 and subsequently detected by the light receiving unit 3 by way of the light receiving optical fiber 19. The intensity of the transmitted light passing through the lubricant 5 decreases as the amount of the foreign matter such as, for example, iron powder (powdery wear debris) contained in the lubricant 5 is large and, accordingly, from the intensity of the transmitted light detected by the light receiving unit 3, the determining circuit 4 can estimate the amount of the foreign matter contained in the lubricant 5.

In the bearing assembly 31 having the lubricant 5 sealed therein, a major cause of deterioration of the lubricant 5 may be an admixture of a powdery wear debris such as, for example, iron powder, which is produced as the bearing assembly 31 is used, into the lubricant 5. Accordingly, by estimating the amount of the foreign matter admixed into the lubricant 5 by the determining circuit 4, the status of deterioration of the lubricant 5 can be estimated. By way of example, in the event that the intensity of the transmitted light detected is lower than a predetermined value, the determining circuit 4 determines that the lubricant is in a contaminated condition.

In particular, since in this lubricant deterioration detecting device 1, the respective ends 18a and 19a of the light emitting and receiving optical fibers 18 and 19 are held in face-to-face relation with the lubricant 5 forming the object to be detected and the other ends 18b and 19b of those optical fibers 18 and 19 are connected respectively with the light emitting unit 2 and the light receiving unit 3, the light emitting unit 2, the light receiving unit 3 and the determining circuit 4 can be arranged separated from the detecting section 20 made up of the respective ends 18a and 19a of the light emitting and receiving optical fibers 18 and 19. For this reason, the light receiving unit 3 and an electronic circuit including the determining circuit 4 or the like can stably detect the status of deterioration of the lubricant 5 without being adversely affected by electrical noises and/or temperature dependent change.

Also, since the detecting section 20 can be constructed compactly, where the use is made to detect the deterioration of the lubricant 5 sealed within, for example, the bearing assembly, only the detecting section 20 can be sufficiently installed inside the bearing assembly and can be arranged simply and compactly. In addition, the detecting section 20 does not constitute a cause of interference with the flow of the lubricant 5 and the lubricant 5 can be stably supplied to the detecting section 20, thus increasing the flexibility of arrangement of the detecting section 20 inside the bearing assembly.

Yet, since the respective ends 18a and 19a of the light emitting and receiving optical fibers 18 and 19 form the detecting section 20 of the lubricant 5, the sectional surface area of a measuring site of the lubricant 5 can be minimized and even the lubricant 5 having a high viscosity can readily enter the detecting section 20, with the detection consequently stabilized.

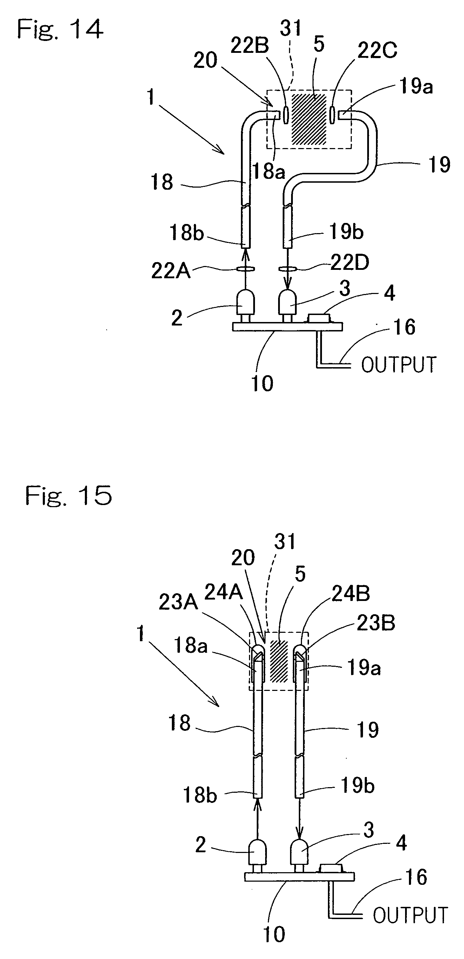

Fig. 14 illustrates a schematic structural diagram showing a ninth preferred embodiment of the present invention. The lubricant deterioration detecting device 1 according to this embodiment is similar to that according to the eighth embodiment shown in and described with reference to Figs. 12 and 13, but differs therefrom in that light collecting lenses 22A and 22B, 22C and 22D are provided in the opposite ends 18a and 18b, 19a and 19b of the light emitting and receiving optical fibers 18 and 19, respectively. It is, however, to be noted that this embodiment may include at least one of the lenses 22A to 22D which is, for example, provided in only one end of one of the optical fibers 18 and 19. Other structural features than those described above are similar to those shown and employed in connection with the eighth embodiment.

As hereinabove described, where the light collecting lenses 22A and 22B, 22C and 22D are provided in the opposite ends 18a and 18b, 19a and 19b of the light emitting and receiving optical fibers 18 and 19, respectively, it is possible to avoid scattering of light and, therefore, not only can the site of measurement on the lubricant 5 be miniaturized, but also the detecting sensitivity can be increased. Even where the lens is provided only a portion of the ends 18a to 19b of the optical fibers 18 and 19, an effect similar to that described above can be obtained to a certain extent.

Fig. 15 illustrates a schematic structural diagram showing a tenth preferred embodiment of the present invention. The lubricant deterioration detecting device 1 according to this embodiment is similar to that according to the eighth embodiment shown in and described with reference to Figs. 12 and 13, but differs therefrom in that the respective ends 18a and 19b of the light emitting and receiving optical fibers 18 and 19, both of which are on the side confronting the lubricant 5, are so arranged as to confront the lubricant 5 through associated mirrors 23A and 23B. More specifically, the end 18a of the light emitting optical fiber 18 is provided with the mirror 23A so that rays of light emitted therefrom can be transmitted across the lubricant 5 after having been deflected at right angles by the mirror 23A. Similarly, the end 19a of the light receiving optical fiber 19 is provided with the mirror 23B so that the rays of light having passed across the lubricant 5 via the mirror 23A can enter the end 19a of the light receiving optical fiber 19 after having been deflected at right angle by the mirror 23B. Those mirrors 23A and 23B are covered by transparent coverings 24A and 24B together with respective ends 18a and 19a of the optical fibers 18 and 19 to avoid contamination of the mirrors 23A and 24B. In such case, the respective ends 18a and 19a of the optical fibers 18 and 19, the mirrors 23A and 23B and the transparent coverings 24A and 24B altogether constitute the detecting section 20.

As hereinabove described, where the respective ends 18a and 19a of the optical fibers 18 and 19 adjacent the lubricant 5 are so positioned as to confront the lubricant through the associated mirrors 23A and 23B, those respective ends 18a and 19a of the optical fibers 18 and 19 are not required to directly confront the lubricant 5. Because of this, when the detecting section 20 is to be fitted in the bearing assembly, the freedom of orientation of the ends 18a and 19a of the optical fibers 18 and 19 increases and, hence, the detecting section 20 can be fitted to, for example, a sealing area of the bearing assembly and/or the detecting section 20 can be fitted while being inserted into the lubricant 5.

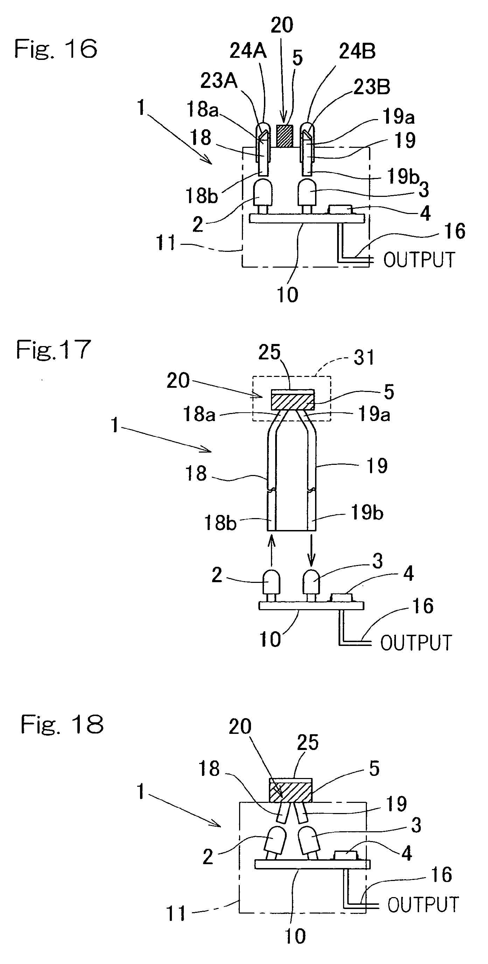

Fig. 16 illustrates a schematic structural diagram showing an eleventh preferred embodiment of the present invention. The lubricant deterioration detecting device 1 according to this embodiment is similar to that according to, for example, the embodiment shown in and described with reference to Fig. 15, but differs therefrom in that the light emitting unit 2, the light receiving unit 3 and the determining circuit 4 are integrated together, having been arranged within, for example, a housing 11 together with the detecting section 20. The detecting section 20 is so provided as to protrude outwardly from the housing 11. Other structural features than those described above are similar to those employed in the embodiment shown in and described with reference to Fig. 15.

Where the light emitting unit 2, the light receiving unit 3 and the determining circuit 4 are integrated together with the detecting section 20 as hereinabove described, on the occasion of detecting the deterioration of the lubricant 5 sealed within the bearing assembly, no wiring work is needed to draw the optical fibers 18 and 19 from inside of the bearing assembly to the outside and installation of the lubricant deterioration detecting device 1 can be facilitated.

Fig. 17 illustrates a schematic structural diagram showing a twelfth preferred embodiment of the present invention. The lubricant deterioration detecting device 1 according to this embodiment is similar to that according to the eighth embodiment shown in and described with reference to Figs. 12 and 13, but differs therefrom in that the respective ends 18a and 19a of the optical fibers 18 and 19 and a reflecting member 25 are so arranged that rays of light emerging outwardly from the end 18a of the light emitting optical fiber 18, which confronts the lubricant 5, can be reflected by the reflecting member 25 before they enter the end 19a of the light receiving optical fiber 19 which confront the lubricant 5. Other structural features than those described above are similar to those employed in the eighth embodiment.

Where as hereinabove described arrangement has been made that the rays of light emerging outwardly from the end 18a of the light emitting optical fiber 18 can enter the end 19a of the light receiving optical fiber 19 after having been reflected by the reflecting member 25, the respective ends 18a and 19a of the optical fibers 18 and 19 need not be so arranged as to confront each other and, accordingly, the freedom of orientation of those ends 18a and 19a can be increased and, hence, the detecting section 20 can be fitted to, for example, a sealing area of the bearing assembly and/or the detecting section 20 can be fitted while being inserted into the lubricant 5.

Fig. 18 illustrates a schematic structural diagram showing a thirteenth preferred embodiment of the present invention. The lubricant deterioration detecting device 1 is similar to that according to the embodiment shown in and described with reference to Fig. 17, but differs therefrom in that the light emitting unit 2, the light receiving unit 3 and the determining circuit 4 are integrated together, having been arranged within, for example, a housing 11 together with the detecting section 20. The reflecting member 25 is suitably arranged separate from the housing 11. Other structural features than those described above are similar to those employed in the embodiment shown in and described with reference to Fig. 17.

Where as hereinabove described the light emitting unit 2, the light receiving unit 3 and the determining circuit 4 are integrated together with the detecting section 20, on the occasion of detecting the deterioration of the lubricant 5 sealed within the bearing assembly, no wiring work is needed to draw the optical fibers 18 and 19 from inside of the bearing assembly to the outside and installation of the lubricant deterioration detecting device 1 can be facilitated.

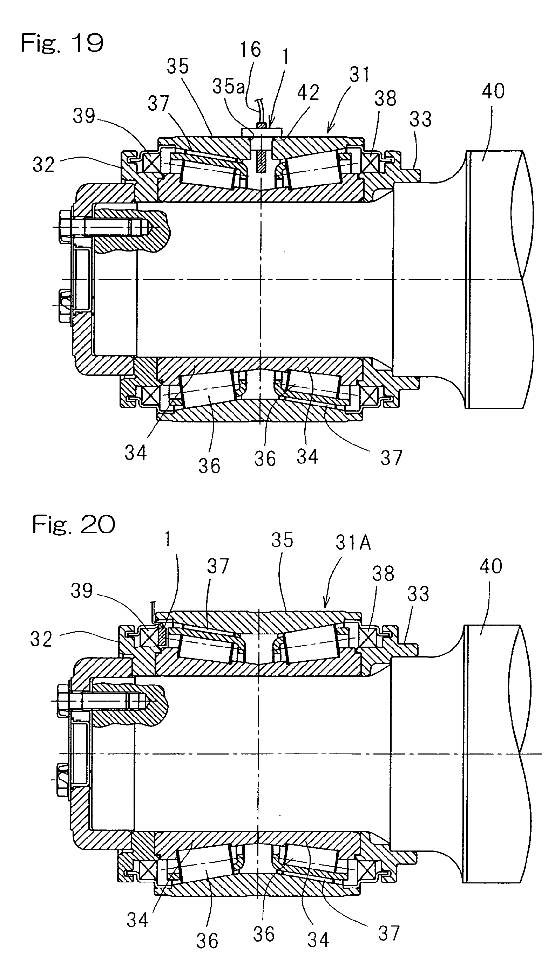

Fig. 19 illustrates a sectional view showing a detecting device incorporated bearing assembly having the lubricant detection detecting device 1 of the structure described hereinabove, which is used in a railway vehicle bearing part. The railway vehicle bearing unit in this case includes a detecting device incorporated bearing assembly 31, and an oil slinger 32 and a rear lid 33, which are accessory components that are provided in contact with opposite ends of an inner ring 34. The bearing assembly 31 is in the form of a rolling bearing, more specifically a double row tapered roller bearing and includes split type inner rings 34 and 34, each provided for a row of rolling elements 36, an unitary outer ring 35, rows of rolling elements 36 and 36 and retainers 37.

The rear lid 33 is mounted on a wheel axle 40 at a location closer to the midpoint thereof than the location of the bearing assembly 31 and has an outer periphery with which an oil seal 38 is slidingly engaged. The oil slinger 32 is mounted on the wheel axle 40 and has an outer periphery with which an oil seal 39 is slidingly engaged. The oil seals 38 and 39 arranged on opposite ends of the bearing assembly 31 serve to seal the lubricant within the bearing assembly 31 and, accordingly, the dust proofing and the resistance to water can be secured.

The lubricant deterioration detecting device I for detecting the status of deterioration of the lubricant sealed within the bearing assembly is mounted on the outer ring 35 of the bearing assembly 31. This lubricant deterioration detecting device 1 is inserted into a detector insertion hole 35a defined in the outer ring 35 at a location intermediate between a plurality of raceway surfaces, with its free end protruding into a bearing space and is fixed in position on the outer ring 35 by means of bolts or the like.

The lubricant deterioration detecting device 1 has the principle construction which may be that according to the first embodiment shown in and described with reference to Figs. 1 to 3 or that according to the second embodiment shown in and described with reference to Fig. 5, but has such an outer appearance as to represents a shape similar to a stepped shaft as shown and is, in its entirety, covered by the housing of this lubricant deterioration detecting device 1. The lubricant deteriorating device 1 and its cable is so treated as to have water and oil resistant properties. A mounting area of the lubricant deterioration detecting device 1 is sealed with an oil resistant material. The lubricant deterioration detecting device 1 is inserted into the detector insertion hole 35a in the outer ring 35 through, for example, a sealing member 42, which may be employed in the form of, for example, an O-ring. As described above, by fixing the lubricant deterioration detecting device 1 on the outer ring 35 through the sealing member 42, it is possible to prevent water and/or dusts from entering into the bearing assembly through the mounting area of the lubricant deterioration detecting device 1.

According to the detecting device incorporated bearing assembly 31 of the construction described hereinabove, since the lubricant deterioration detecting device 1 of the foregoing construction is mounted, deterioration of the lubricant sealed within the bearing assembly can be accurately detected in real time. For this reason, the necessity of replacement of the lubricant can be determined prior to an actual occurrence of an operating abnormality and any damage to the bearing assembly 31 resulting from the failure of the lubricant can be avoided. Also, since the necessity of replacement of the lubricant can be determined in reference to the sensor output, the amount of the lubricant to be discarded earlier than the lifetime of use can be reduced.

Fig. 20 illustrates another example of the detecting device incorporated bearing assembly. This detecting device incorporated bearing assembly 31A is similar to the detecting device incorporated bearing assembly 31 shown in and described with reference to Fig. 19, but differs therefrom in that the lubricant deterioration detecting device 1 is mounted in proximity to the seal 39. Other structural features than those described above are similar to those employed in the example shown in and described with reference to Fig. 19.

It is to be noted that the lubricant deterioration detecting device incorporated bearing assembly according to any one of the foregoing embodiments has been described as applied to the bearing assembly for the railway vehicle, but the lubricant deterioration detecting device incorporated bearing assembly of the present invention can be similarly applied to a bearing assembly for use in an automotive vehicle and an industrial machine and equipment.

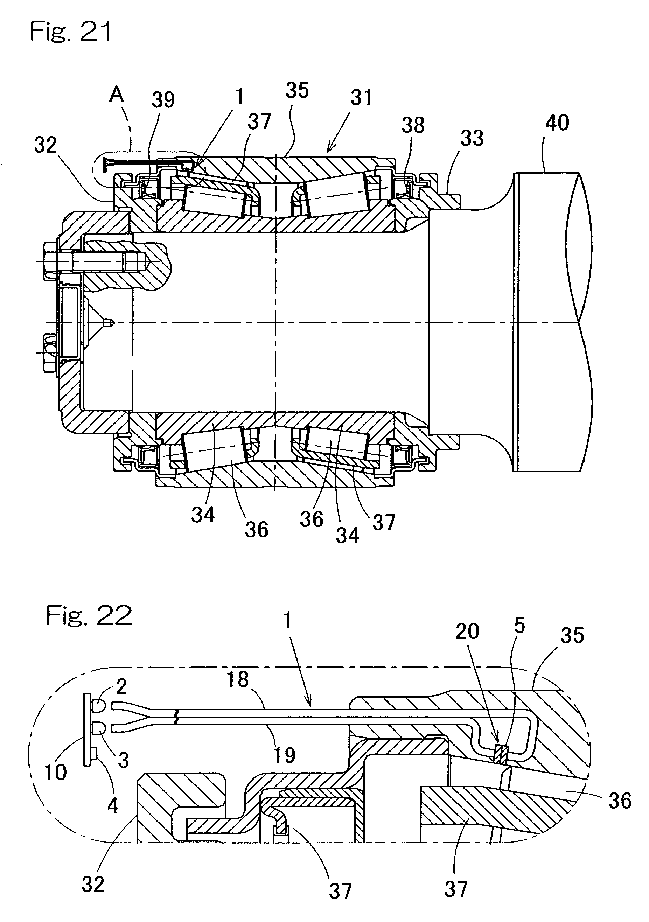

Fig. 21 illustrates a sectional view showing the detecting device incorporated bearing assembly having the lubricant detection detecting device 1 of the structure according to the eighth embodiment shown in and described with reference to Figs. 12 and 13, which is used in a railway vehicle bearing part. The railway vehicle bearing unit in this case includes a detecting device incorporated bearing assembly 31, and an oil slinger 32 and a rear lid 33, which are accessory components that are provided in contact with opposite ends of an inner ring 34. The bearing assembly 31 is in the form of a rolling bearing, more specifically a double row tapered roller bearing and includes split type inner rings 34 and 34, each provided for a row of rolling elements 36, an unitary outer ring 35, rows of rolling elements 36 and 36 and retainers 37.

The rear lid 33 is mounted on a wheel axle 40 at a location closer to the midpoint thereof than the location of the bearing assembly 31 and has an outer periphery with which an oil seal 38 is slidingly engaged. The oil slinger 32 is mounted on the wheel axle 40 and has an outer periphery with which an oil seal 39 is slidingly engaged. The oil seals 38 and 39 arranged on opposite ends of the bearing assembly 31 serve to seal the lubricant within the bearing assembly 31 and, accordingly, the dust proofing and the resistance to water can be secured.

The lubricant deterioration detecting device 1 is fitted to the outer ring 35 and is operable to detect the status of deterioration of the lubricant sealed within the bearing assembly. In such case, as shown in Fig. 22 showing that portion A of Fig. 21 on an enlarged scale, the detecting section 20 of the lubricant deterioration detecting device 1 is arranged on an inner peripheral surface of the outer ring 35, while the light emitting unit 2, the light receiving unit 3 and the determining circuit 4 are arranged outside the bearing assembly. Rays of light emitted from the light emitting unit 2 travel towards the detecting section 20 through the optical fiber 18 which is arranged so as to extend from the detecting section 20 and over the light emitting unit 2, and, hence, the rays of light emerging outwardly from the detecting section 20 reach the light receiving unit 3 through the optical fiber 19 which is arranged so as to extend from the detecting section 20 and over the light receiving unit 3. Insertion of the optical fibers 18 and 19 in the outer ring 35 is treated to have water and oil resistant properties and, accordingly, it is possible to prevent water and/or dusts from entering into the bearing assembly through the mounting area of the lubricant deterioration detecting device 1.

According to the detecting device incorporated bearing assembly 31 having the lubricant deterioration detecting device 1 of the above described construction mounted thereon, deterioration of the lubricant sealed within the bearing assembly can be accurately detected in real time. For this reason, the necessity of replacement of the lubricant can be determined prior to an actual occurrence of an operating abnormality and any damage to the bearing assembly 31 resulting from the failure of the lubricant can be avoided. Also, since the necessity of replacement of the lubricant can be determined in reference to the output of the lubricant deterioration detecting device 1, the amount of the lubricant to be discarded earlier than the lifetime of use can be reduced.

Also, since the light emitting unit, the light receiving unit 3 and the determining circuit 4 are arranged outside the bearing assembly and only the detecting section 20 is arranged in the bearing assembly, the construction of the lubricant deterioration detecting device 1 within the bearing assembly can be downsized.

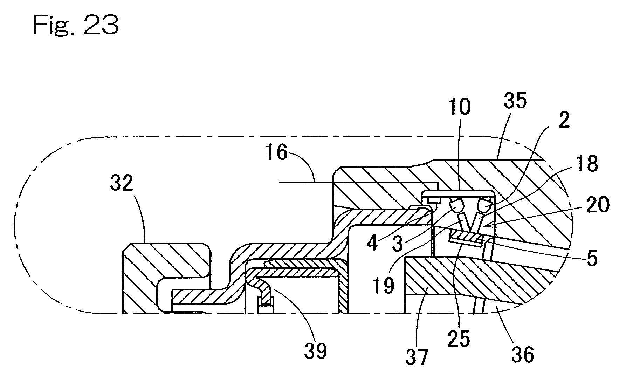

Fig. 23 illustrates a fragmentary enlarged sectional view showing another example of the lubricant deterioration detecting device incorporated bearing assembly. This lubricant deterioration detecting device equipped bearing assembly 31 is similar to the lubricant deterioration detecting device equipped bearing assembly 31 shown in and described with reference to Figs. 21 and 22, but differs therefrom in that the lubricant deterioration detecting device 1 according to the embodiment shown in and described with reference to Fig. 18 is mounted on an inner peripheral surface of the outer ring 35. In this case, since in this lubricant deterioration detecting device 1, the light emitting unit 2, the light receiving unit 3 and the determining circuit 4 are integrated with the detecting section 20, the lubricant deterioration detecting device in its entirety can be snugly and neatly accommodated within the bearing assembly. Only the wiring cable 16 of the lubricant deterioration detecting device 1 is drawn outwardly from inside of the bearing assembly to the outside.

It is to be noted that although in each of the examples of the lubricant deterioration detector incorporated beating assembly 31 shown in Figs. 21 to 23, reference has been made to the lubricant deterioration detecting device 1 according to the eighth embodiment shown in and described with reference to Figs. 12 and 13 and the lubricant deterioration detecting device 1 according to the embodiment shown in and described with reference to Fig. 18 incorporated therein, the lubricant deterioration detecting device 1 according to any one of the embodiments shown in and described with reference to Figs. 14 to 17 may be incorporated therein.

Hereinafter, an applied technology different from the present invention in respect of the basic structure will now be described.

The lubricant deterioration detecting device pertaining to the applied technology different from the present invention in respect of the basic structure includes a first detecting section for measuring a characteristics of a reference lubricant sealed by a sealing member, a second detecting section for measuring a characteristics of a lubricant that is to be detected as to deterioration, and a comparing circuit for comparing respective outputs from the first and second detecting sections to thereby detect the status of deterioration of the lubricant.

According to this construction, as the lubricant forming the object to be detected deteriorates, a deviation will occur between the measurement of the characteristic of the reference lubricant by the first detecting section and the measurement of the characteristic of the lubricant forming the object to be detected by the second detecting section and, hence, the status of deterioration of the lubricant can be detected by the comparing circuit for comparing those measurements. In such case, since the characteristic of the lubricant forming the object to be detected is compared with the characteristic of the reference lubricant, it is possible to detect the deviation in characteristic from the reference lubricant at all times without being affected by a change in measurement environment. Also, since the reference lubricant is prepared merely for detection, by the use of a protecting member such as, for example, a sealing member the status of the respective lubricant is easily maintained with little deterioration. Accordingly, a change of the lubricant as a result of oxidization, component leakage and vaporization can be accurately measured for a long period of time. Accordingly, the status of deterioration of the lubricant forming the object to be detected can be accurately detected.

In the applied technology referred to above, the first and second detecting sections may be of a type capable of measuring an electrical characteristic of the lubricant such as, for example, the dielectric constant, volumetric resistivity or electrostatic capacitance.

Also, the first and second detecting sections may be of a type capable of measuring an optical characteristic of the lubricant such as, for example, transparency or coloring. In the case of the measurement relying on the optical characteristic, the measurement of the difference in degree of deterioration can be easily accomplished with high accuracy as compared with the measurement of the electrical characteristic.

The first and second detecting sections may be juxtaposed on the common circuit substrate. Also, the first and second detecting sections may be arranged on front surface and back surface of the common circuit substrate. When the first and second detecting sections are arranged on the same circuit substrate, the lubricant deterioration detecting device can be constructed compactly and can easily be incorporated into the bearing assembly. In particular where the first and second detecting sections are mounted on front surface and back surface of the common circuit substrate, it can be rendered to be a lubricant deterioration detecting device of a kind, which utilizes the circuit substrate efficiently and is, hence, further compact in size.

Also, in the above described applied technology, electric circuit component parts forming the first and second detecting sections and the comparing circuit may be mounted on the common circuit substrate and the circuit substrate having those electric circuit component parts mounted thereon may be covered with a protecting member having an electrical shielding function and an oil resistance.

Where the lubricant deterioration detecting device is mounted on, for example, a bearing assembly, the circuit component parts may be electrically affected from the periphery and/or may be invaded by, for example, the lubricant as a result of the use thereof for a long period of time, but covering them with the protecting member having the electrical shielding function and the oil resistance is effective to avoid an erroneous operation of the circuit component parts and/or damage from, for example, the lubricant.

The lubricant deterioration detecting device incorporated bearing assembly according to the applied technology different from the present invention with respect to the basic structure is a bearing assembly having mounted thereon the lubricant deterioration detecting device according to the applied technology of the present invention.

According to this construction, features of the lubricant deterioration detecting device according to the applied technology of the present invention can be utilized effectively and the status of deterioration of the lubricant within the bearing assembly can be accurately and in real time detected. Because of this, it is possible to early detect the status of deterioration of the lubricant due to, for example, the detection carried out regularly or in real time, thereby facilitating a predication of any abnormality and/or the timing at which maintenance is performed.

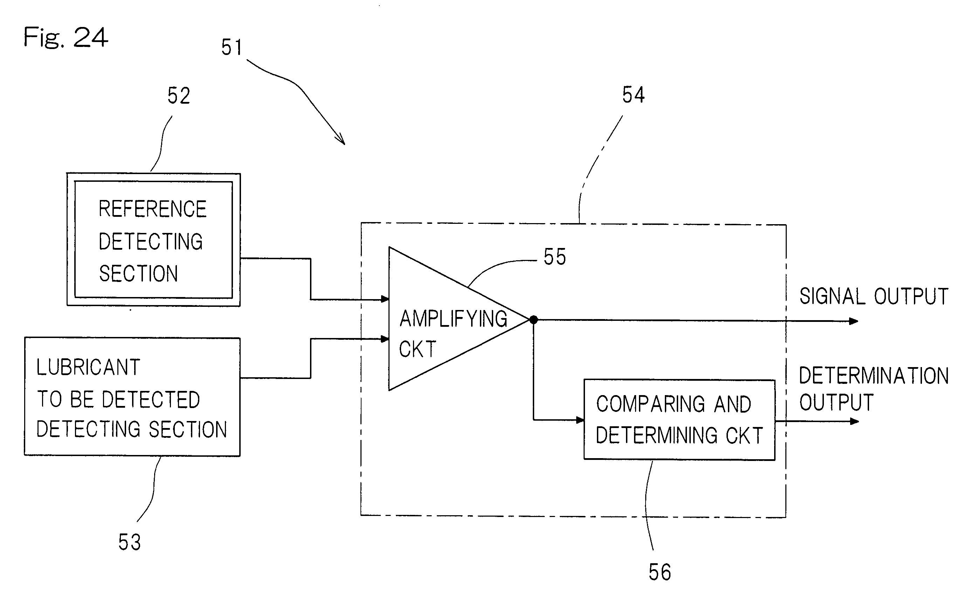

A first specific example according to the above discussed applied technology will be described with particular reference to Figs. 24 to 27. Fig. 24 illustrates a block diagram showing a basic structure of the lubricant deterioration detecting device according to this specific example. This lubricant deterioration detecting device 51 includes a first detecting section 52 for measuring the characteristic of a reference lubricant sealed by a sealing member, a second detecting section 53 for measuring the characteristic of a lubricant forming an object to be detected, and a comparing circuit 54 for comparing respective output signals from the first and second detecting sections 52 and 53 to thereby detect the status of deterioration of the lubricant. The first and second detecting sections 52 and 53 may be of a type capable of measuring an electrical characteristic of the lubricant such as, for example, the dielectric constant, volumetric resistivity or electrostatic capacitance or an optical characteristic of the lubricant such as, for example, transparency or coloring, while in this specific example, they are of the type capable of detecting the electrical characteristic.

The comparing circuit 54 is of a type capable of comparing results of detection of the reference lubricant and the lubricant subject to measurement with each other to thereby evaluate the degree of change of the lubricant to be measured relative to the reference lubricant. In the illustrated instance, the comparing circuit 54 includes a differential amplifying circuit 55 for amplifying and outputting a difference between the detection signal from the first detecting section 52 and the detection signal from the second detecting section 53, and a comparing and determining circuit 56 for comparing an output signal from the differential amplifying circuit 55 with a reference level, which has been predetermined, and subsequently outputting a determined deterioration result. It is to be noted that the comparing circuit 54 may be of a type capable of estimating the status of deterioration from the output signal of the differential amplifying circuit 55. Other than that, the differential detecting construction of the comparing circuit 54 may be formed by a bridge circuit.

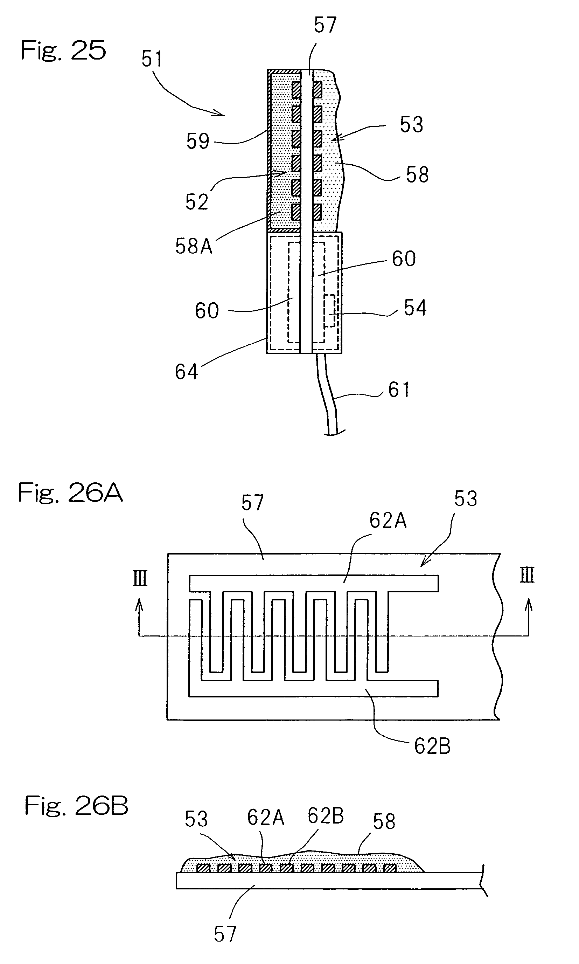

Fig. 25 illustrates a sectional view showing an example of a specific construction where the lubricant deterioration detecting device 51 is so designed as to detect the electrical characteristic. In this figure, the first detecting section 52 is arranged on one surface of a circuit substrate 57 and the second detecting section 53 is arranged on the other surface of the circuit substrate 57. A reference lubricant 58A that is detected by the first detecting section 52 is accommodated within a sealing member in the form of a deterioration protective casing 59, on the side of one surface of the circuit substrate 57, on which the first detecting section 52 is arranged, so as to avoid an entry of foreign matter from the outside and also as to prevent a component from being oxidized and/or vaporized. The second detecting section 53 is exposed to the lubricant 58 forming the object to be detected. Also, the circuit substrate 57 referred to above has electric circuit component parts forming a drive circuit 60 for each of the detecting sections 52 and 53 and those forming the comparing circuit 54 mounted thereon, and those electric circuit component parts are covered by a protecting member 64. The protecting member 64 is made up of a resin portion in the form of a resinous casing or a resin molding having an oil resistance, and an electric shield processing portion (not shown). The electric shield processing portion may be provided in the resin portion referred to above or may be provided separate from a casing or the like. A determining signal from the comparing circuit 54 is outputted to the outside through a wiring cable 61. Also, an electric power is supplied from the outside to the lubricant deterioration detecting device 51 through such wiring cable 61.

Figs. 26A and 26B illustrate one example of the second detecting section 53 designed to detect the dielectric constant or the resistivity or the like. In this example, a pair of comb-shaped electrodes 62A and 62B are mounted on a substrate 57 so as to confront each other to thereby form the second detecting section 53 and are so designed as to measure the electrostatic capacitance or the electric resistivity or the like between those electrodes 62A and 62B. In this way, where the lubricant 58 forming the object to be detected is present on the electrodes 62A and 62B as shown in Fig. 26B, the electric characteristic such as, for example, the dielectric constant, the volumetric resistivity or the electrostatic capacitance can be detected. In this case, although not shown, the first detecting section 52 is of a structure similar to that of the second detecting section 53 and has one surface thereof to which the reference lubricant 58A is sealed by means of the deterioration protective casing 59 shown in Fig. 25.

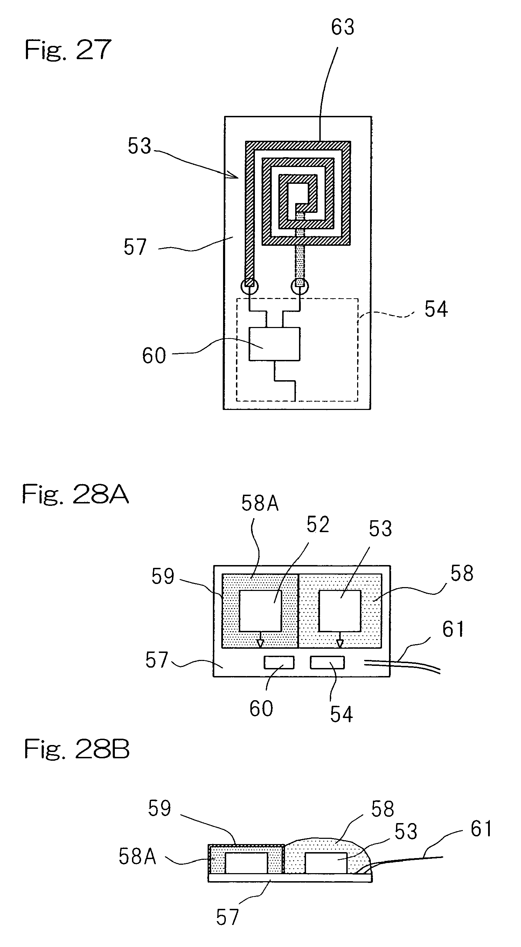

Fig. 27 illustrates an example of the second detecting section 53 that is designed to detect the dielectric constant or the magnetic permeability or the like. In this example, a coil 63 is arranged on a circuit substrate 57 to thereby form the second detecting section 53 for measuring the dielectric constant or the magnetic permeability. In this way, if the lubricant forming the object to be detected is present on the coil 63, the magnetic permeability of the lubricant can be detected. A change in dielectric constant can also be detected. In such case, although not shown, the first detecting section 52 is similar in construction to the second detecting section 53 shown in Fig. 27 and has its surface to which the reference lubricant 58A is sealed by means of the deterioration protective casing 59 shown in Fig. 25.

It is to be noted that in the construction for detecting the magnetic permeability, coaxial placement of the respective coils 63 on front and back surfaces of the circuit substrate 57 will result in an increased interference and, therefore, it is necessary to arrange them in the form as displaced in position.

In the lubricant deterioration detecting device 51 so constructed as hereinabove described, in order to compare the measurement of the reference lubricant 58A (Fig. 25), which has not deteriorated, by means of the first detecting section 52 and the measurement of the lubricant 58 forming the object to be detected by means of the second detecting section 53 with each other, the lubricant deterioration detecting device 51 is so calibrated that an output signal from the differential amplifying circuit 55 may be zero in an initial state. As the lubricant 58 to be detected as to deterioration is deteriorated, a deviation from the measurement of the reference lubricant 58A occurs and a signal indicative of the difference thereof is outputted from the differential amplifying circuit 55. In such case, since the characteristic of the lubricant 58 to be detected as to deterioration is compared with the characteristic of the reference lubricant 58A, which is not deteriorated by oxidization or the like, the deviation from the reference lubricant 58A can be always detected without being affected by a change in measuring environment such as, for example, temperature dependent change. Also, since the status of reference lubricant 58A is maintained with little deterioration, a change of the lubricant 58 resulting from oxidization and component leakage, and a change of the lubricant as a result of vaporization can be measured for a long period of time. Accordingly, the status of deterioration of the lubricant forming the object to be detected can be accurately detected.

It is to be noted that in such case, the reference lubricant 58A and the lubricant 58 forming the object to be detected are preferably arranged in proximity to each other. When so arranged in proximity to each other, both of the lubricants 58A and 58 are similarly affected in the event of occurrence of a change in temperature and/or a change in electric power environment, and, accordingly, such change in temperature and/or such change in electric power environment will bring no influence on the output signal and a stabilized detection of the status of deterioration is possible.

Although the first and second detecting sections 52 and 53 may be arranged on different circuit substrates, arrangement of the first detecting section 52 and the second detecting section 53 on the front and back surfaces of the common circuit substrate 57, respectively, as is the case with this specific example, is effective to construct the lubricant deterioration detecting device 51 compactly and also to facilitate incorporation into the bearing assembly.

Figs. 28A and 28B illustrate a second specific example according to the above described applied technology. The lubricant deterioration detecting device 51 according to this specific example is similar to that according to the first specific example shown in and described with reference to Fig. 24, but differs therefrom in that for the first and second detecting sections 52 and 53, a detecting section for measuring the intensity of the transmitted light is employed. Similarly to the case of the first specific example, a differential detecting system utilizing the reference lubricant 58A is employed. In this specific example, the first and second detecting sections 52 and 53 are arranged juxtaposed on the common circuit substrate 57. Also similarly to the case of the first specific example, the reference lubricant 58A is accommodated in the deterioration protecting casing 59, which is a sealing member, at an area of the surface of the circuit substrate 57 where the first detecting section 52 is arranged, the second detecting section 53 is exposed to the lubricant 58 forming the object to be detected, and the electric circuit component parts of the drive circuit 60 for the first and second detecting sections 52 and 53, and the comparing circuit 54 are mounted on the common circuit substrate 57. It is to be noted that the first and second detecting sections 52 and 53 may be of a type capable of measuring an optical characteristic such as, for example, coloring, other than measuring the transparency.

Even in the case of the lubricant deterioration detecting device 51 according to this specific example, when the detection signal of the first detecting section 52 for measuring the amount of the light transmitted through the reference lubricant 58A and the detection signal of the second detecting section 53 for measuring the amount of the light transmitted through the lubricant 58 forming the object to be detected as to deterioration are compared by the comparing circuit 54, the status of deterioration of the lubricant 58 can be detected accurately.

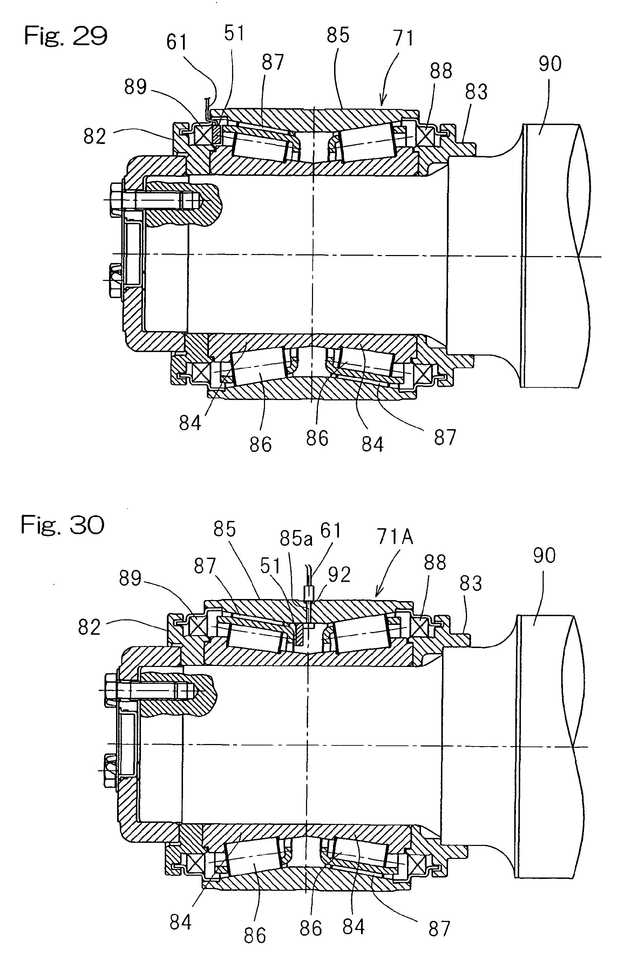

Fig. 29 illustrates a sectional view showing a detecting device incorporated bearing assembly having mounted thereon the lubricant detection detecting device 1 of the structure described hereinabove, which is used in a railway vehicle bearing part. The bearing unit for the railway vehicle in this case includes a detecting device incorporated bearing assembly 71, and an oil slinger 82 and a rear lid 83, which are accessory components that are provided in contact with opposite ends of an inner ring 84. The bearing assembly 71 is in the form of a rolling bearing, more specifically a double row tapered roller bearing and includes split type inner rings 84 and 84, each provided for a row of rolling elements 86, an unitary outer ring 85, rows of rolling elements 86 and 86 and retainers 87.

The rear lid 83 is mounted on a wheel axle 90 at a location closer to the midpoint thereof than the location of the bearing assembly 71 and has an outer periphery with which an oil seal 88 is slidingly engaged. The oil slinger 82 is mounted on the wheel axle 90 and has an outer periphery with which an oil seal 89 is slidingly engaged. The oil seals 88 and 89 arranged on opposite ends of the bearing assembly 71 serve to seal the lubricant within the bearing assembly 71 and, accordingly, the dust proofing and the resistance to water can be secured.

The lubricant deterioration detecting device 51 for detecting the status of deterioration of the lubricant sealed within the bearing assembly is fitted in proximity of the seal 89 of the bearing assembly 71, and the detection signal of the lubricant deterioration detecting device 51 is outputted to the outside through a wiring cable 61. Also, supply of an electric power to the lubricant deterioration detecting device 51 is carried out through the wiring cable 61. An area from which the wiring cable 61 is drawn to the outside is sealed with, for example, an oil resistant resin material to thereby avoid an ingress of water and/or oil leakage.

With the detecting device incorporated bearing assembly 71 having the above described lubricant deterioration detecting device 51 mounted thereon, the deterioration of the lubricant sealed within the bearing assembly can be detected accurately and in real time.

Fig. 30 illustrates a further example of the detecting device incorporated bearing assembly according to the applied technology. This detecting device incorporated bearing assembly 71A is similar to the detecting device incorporated bearing assembly 71 shown in and described with reference to Fig. 29, but differs therefrom in that the lubricant deterioration detecting device 51 is fitted to an inner diametric surface of the outer ring 85 at a location intermediate between the rows of the rolling elements of the bearing assembly 71. The lubricant deterioration detecting device 51 is arranged in proximity to an end face of the rollers 86 or an end face of the retainer 87. The outer ring 85 is formed with a cable insertion hole 85a through which the wiring cable 61 of the lubricant deterioration detecting device 51 is inserted, and a water resistant seal 92 is provided at the position where the wiring cable 61 is inserted. Other structural features than those described above are similar to those in the example shown in and described with reference to Fig. 29.

## Claims

1. A lubricant deterioration detecting device which comprises:
a light emitting unit and a light receiving unit so arranged as to define a gap therebetween for accommodating a lubricant forming an object to be detected; and
a determining circuit for determining a light transmittance of the lubricant in reference to an output from the light receiving unit to thereby detect a status of deterioration of the lubricant.

2. The lubricant deterioration detecting device as claimed in Claim 1, further comprising a set of a light emitting unit and a light receiving unit having a gap therebetween for accommodating a lubricant forming a reference, wherein the determining circuit compares respective outputs of two sets of the light emitting unit and the light receiving unit to thereby detect the status of deterioration of the lubricant.

3. The lubricant deterioration detecting device as claimed in Claim 1, wherein the light emitting unit includes at least one light emitting element and the light receiving unit includes a plurality of light receiving elements, wherein one or some of the plural light receiving elements are arranged in a direction, in which rays of light emitted from the light emitting element and subsequently transmitted through the lubricant forming the object to be detected are detected, and the remaining light receiving elements are arranged in a direction, in which the rays of light emitted from the light emitting element and subsequently scattered by the lubricant are captured, and wherein the determining circuit is operable to compare an output or outputs from the light receiving elements, arranged in the direction for detecting the transmitted rays of light, and the remaining light receiving elements to thereby detect the status of deterioration of the lubricant.

4. The lubricant deterioration detecting device as claimed in Claim 1, wherein the light emitting unit comprises a plurality of light emitting elements operable to emit respective rays of light having different wavelengths and the light receiving unit includes a light receiving elements for receiving rays of light from the plural light emitting elements and wherein the determining circuit is operable to detect the status of deterioration of the lubricant on the basis of a difference in absorptivity for each of the wavelengths detected from an output of the light receiving element.

5. The lubricant deterioration detecting device as claimed in Claim 1, wherein the light emitting unit includes at least one light emitting element and the light receiving unit includes a plurality of light receiving elements having different wavelength sensitivities and capable of receiving rays of light from the light emitting element and wherein the determining circuit is operable to detect the status of deterioration of the lubricant on the basis of a difference in absorptivity for each of the wavelengths detected by comparing respective outputs of the light receiving elements.

6. The lubricant deterioration detecting device as claimed in Claim 1, further comprising a light emitting optical fiber having one end confronting the lubricant and a light receiving optical fiber having one end confronting the lubricant, wherein the light emitting unit and the light receiving unit are connected respectively with other ends of the light emitting optical fiber and receiving optical fiber.

7. The lubricant deterioration detecting device as claimed in Claim 6, wherein one or both of the optical fibers is provided with a lens at one end thereof.

8. The lubricant deterioration detecting device as claimed in Claim 6, wherein the end of one or both of the optical fibers, which in on the side confronting the lubricant, confronts the lubricant via a mirror.

9. A lubricant deterioration detecting device incorporated bearing assembly having the lubricant deterioration detecting device, mounted thereon, as defined in Claim 1.

10. A lubricant deterioration detecting device incorporated bearing assembly having the lubricant deterioration detecting device, mounted thereon, as defined in Claim 6.

11. The lubricant deterioration detecting device incorporated bearing assembly as claimed in Claim 10, wherein the light emitting unit and the light receiving unit are arranged within the bearing assembly.

12. The lubricant deterioration detecting device incorporated bearing assembly as claimed in Claim 10, wherein the light emitting unit and the light receiving unit are arranged outside the bearing assembly.
